# EUROPEAN PATENT APPLICATION

(11) **EP 0 872 233 A1**
(43) Date of publication of application: **21.10.1998**
(21) Application number: 97201100.1
(22) Date of filing: 14.04.1997
(51) Int. Cl.: A61K 9/14, A61K 9/20

(54) **Antiretroviral compositions with improved bioavailability**

(71) Applicant: JANSSEN PHARMACEUTICA N.V., 2340 Beerse (BE)
(72) Inventor: Baert, Lieven Elvire Colette, 2340 Beerse (BE); Verreck, Geert, 2340 Beerse (BE)

(57) **Abstract**

The present invention is concerned with novel pharmaceutical compositions of loviride which can be administered to a patient suffering from a retroviral infection, whereby such dosage forms have a high drug content and can be administered at any time of the day independently of the food taken in by said patient. These novel compositions comprise particles obtainable by melt-extruding a mixture comprising loviride and an appropriate water-soluble polymer and subsequently milling said melt-extruded mixture.

## Description

The present invention is concerned with novel pharmaceutical compositions of loviride which can be administered to a patient suffering from a retroviral infection, whereby such dosage forms have a high drug content and can be administered at any time of the day independently of the food taken in by said patient. These novel compositions comprise particles obtainable by melt-extruding a mixture comprising loviride and an appropriate water-soluble polymer and subsequently milling said melt-extruded mixture.

The development of pharmaceutical compositions having good bioavailability of loviride, a compound that is practically insoluble in aqueous media, remains one of the main challenges of pharmaceutical development of this compound.

The term "practically insoluble" or "insoluble" is to be understood as defined in the United States Pharmacopeia, i.e. a "very slightly soluble" compound requiring from 1000 to 10,000 parts of solvent for 1 part of solute; a "practically insoluble" or "insoluble" compound requiring more than 10,000 parts of solvent for 1 part of solute. The solvent referred to herein is water.

Loviride or (±)-α-[(2-acetyl-5-methylphenyl)amino]-2,6-dichlorobenzeneacetamide, is an antiretroviral non-nucleoside reverse transcriptase inhibitor developed for oral, parenteral and topical administration to patients suffering from HIV infection and is disclosed in WO-92/00952 (23.01.1992). Loviride is currently undergoing extensive phase II evaluations as monotherapy and in combinations with other anti-HIV compounds. As its half-life is short, loviride is admininistered three times a day (t.i.d.). Suitable oral doses are 100 mg t.i.d., 200 mg t.i.d. or even 300 mg t.i.d. The oral formulation can be a capsule comprising drug-coated beads. Since not more than 100 mg of the active ingredient can be formulated into one capsule, patients are expected to ingest from 3 to 9 capsules a day. Clearly, a dosage form having a higher drag content, e.g. 200 mg or even 300 mg would mark a significant step forward.

The term 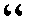 loviride 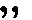 as used hereinafter is to be interpreted broadly and comprises the free base form and the pharmaceutically acceptable addition salts of loviride, or of one of its enantiomers, or of a mixture of its two enantiomers. The preferred loviride compound is the racemic mixture of the enantiomers in the free base form ; to all practical purposes this compound is insoluble. Its solubility at room temperature in water at a pH of 6.5 is less than 0.1 mg/100ml. The acid addition forms may be obtained by reaction of the base form with an appropriate acid. Appropriate acids comprise, for example, inorganic acids such as hydrohalic acids, e.g. hydrochloric or hydrobromic acid; sulfuric acid; nitric acid; phosphoric acid and the like; or organic acids such as, for example, acetic, propanoic, hydroxyacetic, 2-hydroxypropanoic, 2-oxopropanoic, ethanedioic, propanedioic, butanedioic, (Z)-butenedioic, (E)-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids.

In order to achieve the desired antiretroviral effect, it is essential that therapeutically effective plasma levels of loviride can be maintained. As loviride is practically insoluble, effective formulations should be designed in such a manner that the drug is readily bioavailable. In other words, the main problem with the administration of loviride in therapeutically effective amounts is concerned with ensuring that a sufficient amount of loviride remains in a bioavailable physical form (solution, microcrystal) sufficiently long to allow it to get into the circulation, and does not convert into a form that is not readily bioavailable, in particular crystalline loviride (which is formed for example when loviride precipitates in an aqueous medium). To that purpose loviride in capsules is preferably ingested during or at the end of a meal. This, however, limits the ease with which the patients can comply with their prescribed therapy ; for example, some patients are not able to eat normally or swallow medicaments easily (let alone three times a day) because of illness, nausea or because of opportunistic infections of the esophagus. It would therefore be highly desirable to have pharmaceutical dosage forms which have a high drug content and can be administered to a patient at any time of the day independently of food taken in. i.e. dosage forms which can be administered to patients in a fasted state.

The present invention provides pharmaceutical compositions of loviride and a water-soluble polymer which can be administered to a patient suffering from a retroviral infection, whereby such dosage forms can be administered at any time of the day independently of the food taken in by said patient. The bioavailability of the drug from these dosage forms in fasted and in fed patients is comparable. The dosage forms can be prepared easily, for example by conventional tabletting techniques. The dosage forms comprise a therapeutically effective amount of novel particles as described in detail hereunder.

Said novel particles consist of a solid dispersion comprising
(a) loviride, or one of its enantiomers, or a mixture of its two enantiomers; and
(b) one or more pharmaceutically acceptable water-soluble polymers.

The term 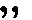 a solid dispersion 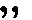 defines a system in a solid state (as opposed to a liquid or gaseous state) comprising at least two components, wherein one component is dispersed more or less evenly throughout the other component or components. When said dispersion of the components is such that the system is chemically and physically uniform or homogenous throughout or consists of one phase (as defined in thermodynamics), such a solid dispersion will be called 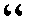 a solid solution 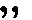 hereinafter. Solid solutions are preferred physical systems because the components therein are usually readily bioavailable to the organisms to which they are administered. This advantage can probably be explained by the ease with which said solid solutions can form liquid solutions when contacted with a liquid medium such as gastric juice. The ease of dissolution may be attributed at least in part to the fact that the energy required for dissolution of the components from a solid solution is less than that required for the dissolution of components from a crystalline solid phase.

The term 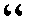 a solid dispersion 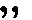 also comprises dispersions which are less homogenous throughout than solid solutions. Such dispersions are not chemically and physically uniform throughout or comprise more than one phase. For example, the term 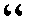 a solid dispersion 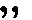 also relates to particles having domains or small regions wherein amorphous or microcrystalline (a), or amorphous or microcrystalline (b), or both, are dispersed more or less evenly in another phase comprising (b), or (a), or a solid solution comprising (a) and (b). Said domains are regions within the particles distinctively marked by some physical feature, small in size compared to the size of the particle as a whole, and evenly and randomly distributed throughout the particle. Microcrystalline (a) typically has a domain size of up to about 25 µm, preferably up to 20 µm.

The particles according to the present invention can be prepared by first preparing a solid dispersion of the components, and then optionally grinding or milling that dispersion. Various techniques exist for preparing solid dispersions including melt-extrusion, spray-drying and solution-evaporation.

The melt-extrusion process comprises the following steps :
a) mixing the components (a) and (b),
b) optionally blending additives with the thus obtained mixture,
c) heating the thus obtained blend until one obtains a homogenous melt,
d) forcing the thus obtained melt through one or more nozzles; and
e) cooling the melt till it solidifies.

The terms "melt" and 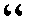 melting 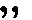 should be interpreted broadly. For our purposes, these terms not only mean the alteration from a solid state to a liquid state, but can also refer to a transition to a glassy state or a rubbery state, and in which it is possible for one component of the mixture to get embedded more or less evenly into the other. In particular cases, one component will melt and the other component(s) will dissolve in the melt thus forming a solution, which upon cooling may form a solid solution having advantageous dissolution properties.

One of the most important parameters of melt extrusion is the temperature at which the melt-extruder is operating. It was found that the operating temperature can easily range between about 120°C and about 300°C. At temperatures lower than 120°C, loviride will not dissolve sufficiently in most water-soluble polymers and the extrudate will not have the required bioavailability. In addition, the process is difficult because of the high viscosity of the mixture. At temperatures of more than 300°C the water-soluble polymer may decompose to an unacceptable level. It may be noted that there is no need to fear decomposition of loviride at temperatures up to 300°C, since this active ingredient is thermally very stable.

The throughput rate is also of importance because even at relatively low temperatures the water-soluble polymer may start to decompose when it remains too long in contact with the heating element.

It will be appreciated that the person skilled in the art will be able to optimize the parameters of the melt extrusion process within the above given ranges. The working temperatures will also be determined by the kind of extruder or the kind of configuration within the extruder that is used. Most of the energy needed to melt, mix and dissolve the components in the extruder can be provided by the heating elements. However, the friction of the material within the extruder may also provide a substantial amount of energy to the mixture and aid in the formation of a homogenous melt of the components.

Spray-drying of a solution of the components also yields a solid dispersion of said components and may be a useful alternative to the melt-extrusion process, particularly in those cases where the water-soluble polymer is not sufficiently stable to withstand the extrusion conditions and where residual solvent can effectively be removed from the solid dispersion. Yet another possibility consists of preparing a solution of the components, pouring said solution onto a large surface, and evaporating the solvent therefrom.

The solid dispersion product is milled or ground to particles having a particle size of less than 600 µm, preferably less than 400 µm and most preferably less than 125 µm. The particle size proves to be an important factor determining the speed with which tablets having sufficient hardness can be manufactured on a large scale. The particle size distribution is such that more than 70% of the particles (measured by weight) have a diameter ranging from about 50 µm to about 500 µm, in particular from about 50 µm to about 200 µm and most in particular from about 50 µm to about 125 µm. Particles of the dimensions mentioned herein can be obtained by sieving them through nominal standard test sieves as described in the CRC Handbook, 64^{th} ed., page F-114. Nominal standard sieves are characterized by the mesh/hole width (µm), DIN 4188 (mm), ASTM E 11-70 (No), Tyler® (mesh) or BS 410 (mesh) standard values. Throughout this description and the claims, particle sizes are designated by reference to the mesh/hole width in µm and to the corresponding Sieve No in the ASTM E11-70 standard.

Preferred are particles wherein the loviride is in a non-crystalline phase as these have an intrinsically faster dissolution rate than those wherein part or all of the loviride is in a microcrystalline form.

Preferably, the solid dispersion is in the form of a solid solution comprising (a) and (b). Alternatively, it may be in the form of a dispersion wherein microcrystalline (a) is dispersed more or less evenly in a solid solution comprising (a) and (b).

The water-soluble polymer in the particles according to the present invention is a polymer that has an apparent viscosity of 1 to 100 mPa.s when dissolved in a 2 % aqueous solution at 20°C solution. For example, the water-soluble polymer can be selected from the group comprising
- alkylcelluloses such as methylcellulose,
- hydroxyalkylcelluloses such as hydroxymethylcellulose, hydroxyethylcellulose, hydroxypropylcellulose and hydroxybutylcellulose,
- hydroxyalkyl alkylcelluloses such as hydroxyethyl methylcellulose and hydroxypropyl methylcellulose,
- carboxyalkylcelluloses such as carboxymethylcellulose,
- alkali metal salts of carboxyalkylcelluloses such as sodium carboxymethylcellulose,
- carboxyalkylalkylcelluloses such as carboxymethylethylcellulose,
- carboxyalkylcellulose esters,
- starches,
- pectines such as sodium carboxymethylamylopectine,
- chitine derivates such as chitosan,
- polysaccharides such as alginic acid, alkali metal and ammonium salts thereof, carrageenans, galactomannans, tragacanth, agar-agar, gummi arabicum, guar gummi and xanthan gummi,
- polyacrylic acids and the salts thereof,
- polymethacrylic acids and the salts thereof, methacrylate copolymers,
- polyvinylalcohol,
- polyvinylpyrrolidone, copolymers of polyvinylpyrrolidone with vinyl acetate,
- polyalkylene oxides such as polyethylene oxide and polypropylene oxide and copolymers of ethylene oxide and propylene oxide.
Non-enumerated polymers which are pharmaceutically acceptable and have appropriate physico-chemical properties as defined hereinbefore are equally suited for preparing particles according to the present invention.

Preferred water-soluble polymers are hydroxypropyl methylcelluloses or HPMC. Said HPMC contains sufficient hydroxypropyl and methoxy groups to render it water-soluble. HPMC having a methoxy degree of substitution from about 0.8 to about 2.5 and a hydroxypropyl molar substitution from about 0.05 to about 3.0 are generally water-soluble. Methoxy degree of substitution refers to the average number of methyl ether groups present per anhydroglucose unit of the cellulose molecule. Hydroxypropyl molar substitution refers to the average number of moles of propylene oxide which have reacted with each anhydroglucose unit of the cellulose molecule. Hydroxypropyl methylcellulose is the United States Adopted Name for hypromellose (see Martindale, The Extra Pharmacopoeia, 29th edition, page 1435). In the four digit number "2910", the first two digits represent the approximate percentage of methoxyl groups and the third and fourth digits the approximate percentage composition of hydroxypropoxyl groups. 5 mPa.s is a value indicative of the apparent viscosity of a 2 % aqueous solution at 20°C.

The molecular weight of the HPMC normally affects both the release profile of the milled extrudate as well as its physical properties. A desired release profile can thus be designed by choosing an HPMC of an appropriate molecular weight ; for immediate release of the active ingredient from the particles, a low molecular weight polymer is preferred. High molecular weight HPMC is more likely to yield a sustained release pharmaceutical dosage form. The molecular weight of a water-soluble cellulose ether is generally expressed in terms of the apparent viscosity at 20°C of an aqueous solution containing two percent by weight of said polymer. Suitable HPMC include those having a viscosity from about 1 to about 100 mPa.s, in particular form about 3 to about 15 mPa.s, preferably about 5 mPa.s The most preferred type of HPMC having a viscosity of 5 mPa.s., is the commercially available HPMC 2910 5 mPa.s, because this yields particles from which superior oral dosage forms of loviride can be prepared as will be discussed hereunder and in the experimental part.

The weight-by-weight ratio of (a) : (b) is in the range of 1 : 1 to 1 : 8, preferably 1 : 1 to 1 : 5. In the case of (loviride) : (HPMC 2910 5 mPa.s), said ratio may range from about 1 : 1 to about 1 : 2, and optimally is about 1 : 1.5 (or 2 : 3). The weight by weight ratio of loviride to other water-soluble polymers may be determined by a person skilled in the art by straightforward experimentation. The lower limit is determined by practical considerations. Indeed, given the therapeutically effective amount of loviride (from about 100 mg to about 300 mg, preferably about 200 mg per administration), the lower limit of the ratio is determined by the maximum amount of mixture that can be processed into one dosage form of practical size. When the relative amount of water-soluble polymer is too high, the absolute amount of mixture needed to reach the therapeutic level will be too high to be processed into one capsule or tablet. Tablets, for example, have a maximum weight of about 1 g, and the extrudate can account for maximally about 90 % (w/w) thereof. Consequently, the lower limit of the amount of loviride over hydroxypropyl methyl cellulose will be about 1 : 8 (100 mg loviride + 800 mg water-soluble polymer).

On the other hand, if the ratio is too high, this means the amount of loviride is relatively high compared to the amount of water-soluble polymer, then there is the risk that the loviride will not dissolve sufficiently in the water-soluble polymer, and thus the required bioavailability will not be obtained. The degree to which a compound has dissolved into a water-soluble polymer can often be checked visually : if the extrudate is clear then it is very likely that the compound will have dissolved completely in the water-soluble polymer. The 1 : 1 upper limit is determined by the fact that above said ratio it was observed that the extrudate resulting from extruding loviride with HPMC 2910 5 mPa.s is not "clear", presumably due to the fact that not all of the loviride has dissolved in the HPMC. It will be appreciated that the upper limit of 1 : 1 may be underestimated for particular water-soluble polymers. Since this can be established easily but for the experimentation time involved, solid dispersions wherein the ratio (a) : (b) is larger than 1 : 1 are also meant to be comprised within the scope of the present invention.

Preferred particles are those obtainable by melt-extrusion of the components and grinding, and optionally sieving. More in particular, the present invention concerns particles consisting of a solid dispersion comprising two parts by weight of loviride and three parts by weight of hydroxypropyl methylcellulose HPMC 2910 5 mPa.s, obtainable by blending said components, extruding the blend at a temperature in the range of 120°C - 300°C, grinding the extrudate, and optionally sieving the thus obtained particles.

The particle as described hereinabove may further comprise one or more pharmaceutically acceptable excipients such as, for example, plasticizers, flavors, colorants, preservatives and the like. Said excipients should not be heat-sensitive, in other words, they should not show any degradation or decomposition at the working temperature of the melt-extruder.

In the current loviride : HPMC 2910 5 mPa.s formulations, the amount of plasticizer is preferably small, in the order of 0 % to 15 % (w/w), preferably less than 5 % (w/w). With other water-soluble polymers though, plasticizers may be employed in much different, often higher amounts because plasticizers as mentioned hereinbelow lower the temperature at which a melt of (a), (b) and plasticizer is formed, and this lowering of the melting point is advantagous where the polymer has limited thermal stability. Suitable plasticizers are pharmaceutically acceptable and include low molecular weight polyalcohols such as ethylene glycol, propylene glycol, 1,2 butylene glycol, 2,3-butylene glycol, styrene glycol; polyethylene glycols such as diethylene glycol, triethylene glycol, tetraethylene glycol; other polyethylene glycols having a molecular weight lower than 1,000 g/mol; polypropylene glycols having a molcular weight lower than 200 g/mol; glycol ethers such as monopropylene glycol monoisopropyl ether; propylene glycol monoethyl ether; diethylene glycol monoethyl ether; ester type plasticizers such as sorbitol lactate, ethyl lactate, butyl lactate, ethyl glycolate, allyl glycollate; and amines such as monoethanolamine, diethanolamine, triethanolamine, monoisopropanolamine; triethylenetetramine, 2-amino-2-methyl-1,3-propanediol and the like. Of these, the low molecular weight polyethylene glycols, ethylene glycol, low molecular weight polypropylene glycols and especially propylene glycol are preferred.

Once the extrudate is obtained, it is milled and sieved and used as a "normal" ingredient to make pharmaceutical dosage forms.

The particles of the present invention can be formulated into pharmaceutical dosage forms comprising a therapeutically effective amount of particles. Although, at first instance, pharmaceutical dosage forms for oral administration such as tablets and capsules are envisaged, the particles of the present invention can also be used to prepare pharmaceutical dosage forms e.g. for rectal administration. Preferred dosage forms are those adapted for oral administration shaped as a tablet. They can be produced by conventional tabletting techniques with conventional ingredients or excipients and with conventional tabletting machines. In addition, they can be produced at substantially lower cost than coated cores. As mentioned above, an effective antiretroviral daily dose of loviride ranges from about 100 mg t.i.d. to about 300 mg t.i.d., and preferably is about 200 mg t.i.d. When one considers that the weight-by-weight ratio of (a) : (b) is maximally about 1 : 1, then it follows that one dosage form will weigh at least 400 mg. In order to facilitate the swallowing of such a dosage form by a patient, it is advantageous to give the dosage form, in particular tablets, an appropriate shape. Tablets that can be swallowed comfortably are therefore preferably elongated rather than round in shape. Especially preferred are biconvex oblate tablets. As discussed hereunder in more detail, a film coat on the tablet further contributes to the ease with which it can be swallowed.

Tablets that give an immediate release of loviride upon oral ingestion and that have good bioavailability are designed in such a manner that the tablets disintegrate rapidly in the stomach (immediate release) and that the particles which are liberated thereby are kept away from one another so that they do not coalesce and do not produce high local concentrations of loviride with the concommittant danger that the drug precipitates (bioavailability). The desired effect can be obtained by distributing said particles homogeneously throughout a mixture of a disintegrant and a diluent.

Suitable disintegrants are those that have a large coefficient of expansion. Examples thereof are hydrophilic, insoluble or poorly water-soluble crosslinked polymers such as crospovidone (crosslinked polyvinylpyrrolidone) and croscarmellose. The amount of disintegrant in immediate release tablets according to the present invention conveniently may range from about 3 to about 15 % (w/w) and preferably is about 7 to 9 % (w/w). This amount tends to be larger than usual in order to ensure that the particles are spread over a large volume of the stomach contents upon ingestion. Because disintegrants by their nature yield sustained release formulations when employed in bulk, it is advantageous to dilute them with an inert substance called a diluent or filler.

A variety of materials may be used as diluents or fillers. Examples are spray-dried or anhydrous lactose, sucrose, dextrose, mannitol, sorbitol, starch, cellulose (e.g. micro-crystalline cellulose Avicel™), dihydrated or anhydrous dibasic calcium phosphate, and others known in the art, and mixtures thereof. Preferred is a commercial spray-dried mixture of lactose monohydrate (75 %) with microcrystalline cellulose (25 %) which is commercially available as Microcelac™.

The tablet may include a variety of one or more other conventional excipients such as binders, buffering agents, lubricants, glidants, thickening agents, sweetening agents, flavors, and colors. Some excipients can serve multiple purposes.

Lubricants and glidants can be employed in the manufacture of certain dosage forms, and will usually be employed when producing tablets. Examples of lubricants and glidants are hydrogenated vegetable oils, e.g hydrogenated Cottonseed oil, magnesium stearate, stearic acid, sodium lauryl sulfate, magnesium lauryl sulfate, colloidal silica, talc, mixtures thereof, and others known in the art. Interesting lubricants and glidants are magnesium stearate, and mixtures of magnesium stearate with colloidal silica. A preferred lubricant is hydrogenated vegetable oil type I, most preferably hydrogenated, deodorized Cottonseed oil (commercially available from Karlshamns as Akofine NF™ (formerly called Sterotex™)). Lubricants and glidants generally comprise 0.2 to 7.0 % of the total tablet weight.

Other excipients such as coloring agents and pigments may also be added to the tablets of the present invention. Coloring agents and pigments include titanium dioxide and dyes suitable for food. A coloring agent is an optional ingredient in the tablet of the present invention, but when used the coloring agent can be present in an amount up to 3.5 % based on the total tablet weight.

Flavors are optional in the composition and may be chosen from synthetic flavor oils and flavoring aromatics or natural oils, extracts from plants leaves, flowers, fruits and so forth and combinations thereof. These may include cinnamon oil, oil of wintergreen, peppermint oils, bay oil, anise oil, eucalyptus, thyme oil. Also useful as flavors are vanilla, citrus oil, including lemon , orange, grape, lime and grapefruit, and fruit essences, including apple, banana, pear, peach, strawberry, raspberry, cherry, plum, pineapple, apricot and so forth, The amount of flavor may depend on a number of factors including the organoleptic effect desired. Generally the flavor will be present in an amount from about 0 % to about 3 % (w/w).

As known in the art, tablet blends may be dry-granulated or wet-granulated before tabletting. The tabletting process itself is otherwise standard and readily practised by forming a tablet from desired blend or mixture of ingredients into the appropriate shape using a conventional tablet press.

Tablets of the present invention may further be film-coated to improve taste, to provide ease of swallowing and an elegant appearance. Many polymeric film-coating materials are known in the art. A preferred film-coating material is hydroxypropyl methylcellulose HPMC, especially HPMC 2910 5 mPa.s. Other suitable film-forming polymers also may be used herein, including, hydroxypropylcellulose, and acrylatemethacrylate copolymers. Besides a film-forming polymer, the film coat may further comprise a plasticizer (e.g. propylene glycol) and optionally a pigment (e.g. titanium dioxide). The film-coating suspension also may contain tale as an anti-adhesive. In immediate release tablets according to the invention, the film coat is small and in terms of weight accounts for about 3 % (w/w) of the total tablet weight.

Preferred dosage forms are those wherein the weight of the particles is at least 40 % of the total weight of the total dosage form, that of the diluent ranges from 20 to 40 %, and that of the disintegrant ranges from 3 to 10 %, the remainder being accounted for by one or more of the excipients described hereinabove. As an example of an oral dosage form comprising 200 mg of loviride, the following formula may be given :
loviride (200 mg)
HPMC 2910 5 mPa.s (300 mg)
spray-dried lactose monohydrate : microcrystalline cellulose (75 : 25) (282.4 mg)
crospolyvidone (78.4 mg)
talc (25.8 mg)
hydrogenated vegetable oil Type I (8.6 mg)
colloidal anhydrous silica (2.6 mg)
magnesium stearate (2.2 mg), yielding
a tablet core (900 mg), and
HPMC 2910 5 mPa.s (16 mg)
propyleneglycol (4 mg)
talc (3.2 mg)
titanium dioxide (4.8 mg), yielding
a film-coat (28 mg).

Preferred dosage forms according to the present invention are those from which at least 85 % of the available loviride dissolves within 60 minutes when a dosage form equivalent to 200 mg loviride is tested as set forth in USP test 〈711〉 in a USP-2 dissolution apparatus under conditions at least as stringent as the following : 900 ml water comprising 0.5 % sodium lauryl sulfate, 37°C with paddles turning at 100 rpm. Tablets complying with the preceding definition can be said to have Q > 85 % (60'). Preferably, tablets according to the present invention will dissolve faster and have Q > 85 % (30').

The present invention further concerns a process of preparing particles as described hereinbefore, characterized by blending the components, extruding said blend at a temperature in the range of 120 - 300 °C, grinding the extrudate, and optionally sieving the particles.

The invention also concerns solid dispersions obtained by melt-extrusion of
(a) loviride, or one of its enantiomers, or a mixture of its two enantiomers, and
(b) one or more pharmaceutically acceptable water-soluble polymers.

It is another object of the invention to provide a process of preparing a pharmaceutical dosage form as described hereinbefore, characterized by blending a therapeutically effective amount of particles as described hereinbefore, with pharmaceutically acceptable excipients and compressing said blend into tablets.

The invention also relates to particles as described hereinbefore, for use in preparing a pharmaceutical dosage form for oral administration to a patient suffering from a retroviral infection, wherein said dosage form can be administered at any time of the day independently of the food taken in by said patient.

The present invention also concerns the use of particles as described hereinbefore, for the preparation of a pharmaceutical dosage form for oral administration to a patient suffering from a retroviral infection, wherein said dosage form can be administered at any time of the day independently of the food taken in by said patient.

The invention also relates to a pharmaceutical package suitable for commercial sale comprising a container, an oral dosage form of loviride as described hereinbefore, and associated with said package written matter non-limited as to whether the dosage form can be taken with or without food.

It has been observed that the tablets of the present invention showed a remarkably lower food-effect than loviride capsules. This means that the difference between taking the medication after a meal or in fasted state is significantly less when the tablet of the present invention is administered than when loviride capsules are administered. This is of course a huge advantage because the medication can be taken in at any time during the day and is no longer dependent upon the intake of a meal. Moreover, patients, who are feeling nauseous or who are not able to eat can still take the tablets of the present invention.

### Example 1

### a) melt extrusion process

A 40/60 (w/w) mixture of loviride (0.5 kg) and HPMC 2910 5 mPa.s (0.75 kg) were both sieved and mixed in a planetary mixer until the mixture was homogenous.

The mixture was fed into a twin screw melt extruder of the type APV-Baker MP19 L/D 15 having the following operating parameters : temperature of the first compartment was 225°C, temperature of the second compartment was 235°C, the twin screw had a rate of 250 revolutions/min and was extruded at a fedd rate of 1.5 kg/h. The extrudate was brought in a hammer mill of type Fitzmill, the mesh of the sieve was 0.125 inch (= 0.32 cm) and revolving speed was 1640 revolutions per minute. The milled extrudate was again brought in a hammer mill, this time with a sieve of mesh 0.063 inch (= 0.16 cm) and a revolving speed of 1640 revolutions per minute. Yield was 1.03 kg (82.4 %).

### b) preparation of a tabletting mixture

Microcrystalline cellulose (90.2 g, 24.4 % (w/w)), Crospovidone (25 g, 6.8 % (w/w)), Aerosil (colloidal silicon dioxide) (1.1 g, 0.3 % (w/w)) and Sterotex (3.7 g, 1 % (w/w)) were sieved (mesh width of 850 µm) and mixed together with the milled extrudate (250 g, 67.6 % (w/w)) using a planetary mixer until a homogenous mixture was obtained (10 minutes).

### c) Tabletting

Using the mixture obtained in b) round biconvex tablets of 370 mg (die diameter = 10 mm, radius of curvature = 15 mm) were prepared on a Korsch II operating at a speed of 10,000 tablets/hour, a compression pressure of 1500 to 1950 kg /cm² (147 - 191.1 MPa). The uncoated tablets had a disintegration time of less than 1 minute in neutral water and hardness = 6.23 daN. The tablets were coated with a coating solution comprising HPMC 2910 5 mPa.s (40 g), propylene glycol (10 g), titanium dioxide (12 g) and tale (8 g) in water (400 g) according to art-known procedures.

### Example 2

The process as described in example 1 was repeated, but the blending and tabletting steps were carried out as follows :

Microcelac (141.2 g, 31.4 % (w/w)), Crospovidone (39.2 g, 8.7 % (w/w)) and Aerosil (colloidal silicon dioxide) (1.3 g, 0.3 % (w/w)) were sieved (mesh width of 850 µm) and mixed together with the milled extrudate (250 g, 55.6 % (w/w)) using a planetary mixer until a homogenous mixture was obtained (10 minutes). Then there was added tale (12.9 g, 2.9 % (w/w)), magnesium stearate (1.1 g, 0.24 % (w/w)) and Sterotex (4.3 g, 1 % (w/w)) and the whole was mixed for another 5 minutes. The blend was tabletted on a Korsch II to round bieonvex tablets of 450 mg (die diameter 11.5 mm, radius of curvature = 15 mm). The tablets were coated with a coating solution comprising HPMC 2910 5 mPa.s (40 g), propylene glycol (10 g), titanium dioxide (12 g) and talc (8 g) in water (400 g) according to art-known procedures.

### Example 3

The process as described in example 1 was repeated, but the blending and tabletting steps were carried out as follows :

Microcelac (282.4 g, 31.4 % (w/w)), Crospovidone (78.4 g, 8.7 % (w/w)) and Aerosil (colloidal silicon dioxide) (2.6 g, 0.3 % (w/w)) were sieved (mesh width of 850 µm) and mixed together with the milled extrudate (500 g, 55.6 % (w/w)) using a planetary mixer until a homogenous mixture was obtained (10 minutes). Then there was added tale (25.8 g, 2.9 % (w/w)), magnesium stearate (2.2 g, 0.24 % (w/w)) and Sterotex (8.6 g, 1 % (w/w)) and the whole was mixed for another 5 minutes. The blend was tabletted on a Excenterpress Courtoy 27 to oblate biconvex tablets of 900 mg. The length of the die was 19 mm, breadth 9.5 mm, and the radius of curvature 9.57 mm. The tablets were coated with a coating solution comprising HPMC 2910 5 mPa.s (40 g), propylene glycol (10 g), titanium dioxide (12 g) and talc (8 g) in water (400 g) according to art-known procedures.

### Example 4 : Dissolution Properties

*In-vitro* dissolutions studies were performed on the 100 mg tablet formulation of Example 1. The medium was 900 ml water comprising 0.5 % sodium lauryl sulfate at 37°C in Apparatus 2 (USP 23, 〈711〉 Dissolution, pp. 1791-1793) (paddle, 100 rpm). The concentration of the active ingredient loviride dissolved in the test medium was determined by removing a 3 ml sample at the indicated time, measuring its absorbance at 266 nm and calculating the concentration therefrom.
The following results were obtained :

| Time (min) | Calculated concentration (% w/w) of the active dose | | | | | | |
|---|---|---|---|---|---|---|---|
| | sample 1 | sample 2 | sample 3 | sample 4 | sample 5 | sample 6 | average |
| 0 | 0.00 | 0.00 | 0.00 | 0.00 | 0.00 | 0.00 | 0.00 |
| 10 | 84.1 | 66.3 | 82.3 | 83.7 | 80.5 | 82.4 | 79.9 |
| 30 | 100.4 | 96 | 100.0 | 101.5 | 97.4 | 97.1 | 98.8 |
| 45 | 102.6 | 102.6 | 103.1 | 103.9 | 100.0 | 99.8 | 102.0 |
| 60 | 102.1 | 102.3 | 103.0 | 103.3 | 99.8 | 99.7 | 101.7 |
| 90 | 102.9 | 103.6 | 104.0 | 104.1 | 100.7 | 99.9 | 102.5 |

### Example 5 : Pharmacokinetic properties

In this study 12 healthy volunteers in fasting conditions were administered a tablet of example 1 (100 mg loviride). Plasma levels of each of the loviride enantiomers were determined and the following bioavailability data were calculated therefrom.
- (-)-loviride :: Cₘₐₓ = 155 ± 66 ng/ml
AUCₗₐₛₜ = 1,347 ± 512 ng.h/ml
- (+)-loviride :: Cₘₐₓ = 890 ± 292 ng/ml
AUCₗₐₛₜ = 18,180 ± 7,197 ng.h/ml

## Claims

1. A particle consisting of a solid dispersion comprising
(a) loviride, or one of its enantiomers, or a mixture of its two enantiomers, and
(b) one or more pharmaceutically acceptable water-soluble polymers.

2. A particle according to claim 1 having a particle size of less than 600 µm.

3. A particle according to claim 1 or 2 wherein the loviride is in a non-crystalline phase.

4. A particle according to claim 3 wherein the solid dispersion is in the form of a solid solution comprising (a) and (b), or in the form of a dispersion wherein amorphous or microcrystalline (a), or amorphous or microcrystalline (b), or both, are dispersed more or less evenly in another phase of (b), or of (a), or in a solid solution comprising (a) and (b).

5. A particle according to the preceding claims wherein the water-soluble polymer is a polymer that has an apparent viscosity of 1 to 100 mPa.s when dissolved in a 2 % aqueous solution at 20°C solution.

6. A particle according to claim 5 wherein the water-soluble polymer is selected from the group comprising
- alkylcelluloses such as methylcellulose,
- hydroxyalkylcelluloses such as hydroxymethylcellulose, hydroxyethylcellulose,
- hydroxypropylcellulose and hydroxybutylcellulose,
- hydroxyalkyl alkylcelluloses such as hydroxyethyl methylcellulose and hydroxypropyl methylcellulose,
- carboxyalkylcelluloses such as carboxymethylcellulose,
- alkali metal salts of carboxyalkylcelluloses such as sodium carboxymethylcellulose,
- carboxyalkylalkylcelluloses such as carboxymethylethylcellulose,
- carboxyalkylcellulose esters,
- starches,
- pectines such as sodium carboxymethylamylopectine,
- chitine derivates such as chitosan,
- polysaccharides such as alginic acid, alkali metal and ammonium salts thereof, carrageenans, galactomannans, tragacanth, agar-agar, gummi arabicum, guar gummi and xanthan gummi,
- polyacrylic acids and the salts thereof,
- polymethacrylic acids and the salts thereof, methacrylate copolymers,
- polyvinylalcohol,
- polyvinylpyrrolidone, copolymers of polyvinylpyrrolidone with vinyl acetate
- polyalkylene oxides such as polyethylene oxide and polypropylene oxide and copolymers of ethylene oxide and propylene oxide.

7. A particle according to claim 6 wherein the water-soluble polymer is hydroxypropyl methylcellulose HPMC 2910 5 mPa.s.

8. A particle according to claim 7 wherein the weight-by-weight ratio of (a) : (b) is in the range of 1 : 1 to 1 : 8.

9. A particle according to any one of the preceding claims obtainable by melt-extrusion of the components and grinding, and optionally sieving.

10. A particle according to any one of the previous claims consisting of a solid dispersion comprising two parts by weight of loviride and three parts by weight of hydroxypropyl methylcellulose HPMC 2910 5 mPa.s, obtainable by blending said components, extrading the blend at a temperature in the range of 120°C - 300°C, grinding the extrudate, and optionally sieving the thus obtained particles.

11. A particle according to the preceding claims further comprising one or more pharmaceutically acceptable excipients.

12. A pharmaceutical dosage form comprising a therapeutically effective amount of particles as claimed in any one of the preceding claims.

13. A dosage form according to claim 12 adapted for oral administration shaped as a tablet.

14. A dosage form according to claim 12 for immediate release of loviride upon oral ingestion wherein said particles are homogeneously distributed throughout a mixture of a diluent and a disintegrant.

15. A dosage form according to claim 13 or 14 surrounded by a film-coat comprising a film-forming polymer, a plasticizer and optionally a pigment.

16. A dosage form according to claim 14 wherein the diluent is a spray-dried mixture of lactose monohydrate and microcrystalline cellulose (75 : 25), and the disintegrant is crospovidone or croscarmellose.

17. A dosage form according to any one of claims 12 to 16 wherein the weight of said particles is at least 40 % of the total weight of the dosage form.

18. A dosage form according to claim 12 comprising by weight based on the total weight of the dosage form :
loviride (200 mg)
HPMC 2910 5 mPa.s (300 mg)
spray-dried lactose monohydrate : microcrystalline cellulose (75 : 25) (282.4 mg)
crospolyvidone (78.4 mg)
talc (25.8 mg)
hydrogenated vegetable oil Type I (8.6 mg)
colloidal anhydrous silica (2.6 mg)
magnesium stearate (2.2 mg), yielding
a tablet core (900 mg), and
HPMC 2910 5 mPa.s (16 mg)
propyleneglycol (4 mg)
talc (3.2 mg)
titanium dioxide (4.8 mg), yielding
a film-coat (28 mg).

19. A dosage form according to any one of claims 12 to 18 from which at least 85 % of the available loviride dissolves within 60 minutes when a dosage form equivalent to 200 mg loviride is tested as set forth in USP test 〈711〉 in a USP-2 dissolution apparatus under conditions at least as stringent as the following : 900 ml water comprising 0.5 % sodium lauryl sulfate, 37°C with paddles turning at 100 rpm.

20. A process of preparing particles as claimed in any one of claims 1 to 11 characterized by blending the components, extruding said blend at a temperature in the range of 120 - 300 °C, grinding the extrudate, and optionally sieving the particles.

21. A solid dispersion obtained by melt-extrusion of
(a) loviride, or one of its enantiomers, or a mixture of its two enantiomers, and
(b) one or more pharmaceutically acceptable water-soluble polymers.

22. A process of preparing a pharmaceutical dosage form as claimed in any one of claims 12 to 19 characterized by blending a therapeutically effective amount of particles as claimed in any one of claims 1 to 11 with pharmaceutically acceptable excipients and compressing said blend into tablets.

23. Particles according to any one of claims 1 to 11 for use in preparing a pharmaceutical dosage form for oral administration to a patient suffering from a retroviral infection, wherein said dosage form can be administered at any time of the day independently of the food taken in by said patient.

24. Use of particles according to any one of claims 1 to 11 for the preparation of a pharmaceutical dosage form for oral administration to a patient suffering from a retroviral infection, wherein said dosage form can be administered at any time of the day independently of the food taken in by said patient.

25. A pharmaceutical package suitable for commercial sale comprising a container, an oral dosage form of loviride as claimed in any one of claims 12 to 19, and associated with said package written matter non-limited as to whether the dosage form can be taken with or without food.
